# EUROPEAN PATENT SPECIFICATION

(11) **EP 3 508 147 B1**
(45) Date of publication and mention of the grant of the patent: **23.04.2025**
(21) Application number: 17845220.7
(22) Date of filing: 16.08.2017
(51) Int. Cl.: A61B 17/32, A61B 17/16, A61B 17/14

(54) **ULTRASONIC OSTEOTOME TOOL BIT**
BOHRSPITZE FÜR ULTRASCHALLOSTEOTOMINSTRUMENT
OUTIL DE COUPE D'OSTÉOTOME À ULTRASONS

(30) Priority: 31.08.2016 CN 201621022534 U
(43) Date of publication of application: 10.07.2019
(73) Proprietor: Jiangsu SMTP Technology Co., Ltd., Zhangjiagang, Jiangsu 215634 (CN)
(72) Inventor: SUN, Xianze, Zhangjiagang Jiangsu 215634 (CN); ZHAN, Songtao, Zhangjiagang Jiangsu 215634 (CN); FENG, Zhen, Zhangjiagang Jiangsu 215634 (CN); CAO, Qun, Zhangjiagang Jiangsu 215634 (CN)
(74) Representative: Daub, Thomas
(86) International application number: PCT/CN2017/097689
(87) International publication number: WO 2018/040918

(56) References cited:
- EP-A1- 1 736 107
- EP-A1- 2 848 214
- CN-U- 202 920 294
- CN-U- 204 133 550
- CN-U- 204 133 551
- CN-U- 205 234 577
- CN-U- 205 234 577
- CN-U- 206 424 121
- US-A- 4 069 824
- US-A- 5 318 570
- US-A1- 2002 042 998
- US-A1- 2007 088 361
- US-A1- 2015 182 232

## Description

### TECHNICAL FIELD

The present disclosure relates to the field of medical instruments and devices, and in particular to an ultrasonic scalpel, and more particularly to a tool bit for an ultrasonic osteotome.

### BACKGROUND

In modern society, with the development of medical technology, orthopedic surgery shows a trend of diversity. Accordingly, in orthopedic surgery, ultrasonic osteotomes are often used to perform cutting, grinding, planing, scraping or arbitrary shaping on bones.

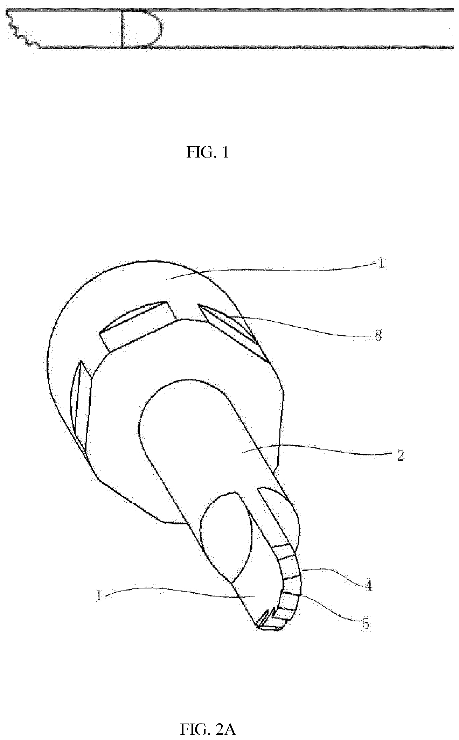

In view of the special construction of bone structure, along with the continuously development of ultrasound technology in recent years, ultrasonic osteotomes have gradually become main tools for modern orthopedic surgery. In the orthopedic surgery, a commonly used ultrasonic osteotome has a sheet-shaped tool bit, which is mainly used for cutting, as shown in Fig. 1. However, at present, these tool bits mostly have a straight sheet shape. When it is necessary to cut a hole in a bone tissue, the straight sheet tool bit has poor operability, and there is a risk of breaking the tool bit when it is used improperly, which can cause damage to other non-surgical parts and thus cause danger. This puts higher requirements on the operation level of the medical staff, places physical and mental pressure on the doctors, reduces the success rate of the operation, and increases the risk of surgery for the patient.

EP 2848214A1 discloses a tool for cutting bone, which comprises an elongate blade connectable to a generator of longitudinal-mode ultrasonic vibrations. The blade has two lateral cutting edges linked by a rounded distal tip. A series of triangular teeth extends along each cutting edge and the distal tip. The blade may taper towards each cutting edge and the distal tip. A variant of the tool comprises an elongate part-cylindrical blade connectable to a generator of torsional-mode ultrasonic vibrations. The blade has a cutting edge at its distal tip provided with a plurality of triangular teeth. All forms of the tool are particularly suitable for cutting cancellous bone around an implant to be removed during revision of a joint arthroplasty.

US 5318570A discloses a mechanical junction for facilitating the rapid attachment and removal of ultrasonic surgical components for the transfer of ultrasonic energy across the junction from an ultrasonic transducer to an ultrasonically activated tool bit. The junction achieves a high, evenly distributed compressive force to optimize propagation of the ultrasonic energy from the transducer to a tool bit, while maintaining a relatively small outside diameter of the ultrasonic tool. Also disclosed are a plurality of ultrasonic energy activated tool tips for introducing and removing an orthopedic prosthesis.

CN205234577U discloses an ultrasonic bone cutter head, which comprises a bit body connected to an ultrasonic device, a bit bar fixed to the bit body, and a flat head fixed to the bit bar. The head includes a tooth edge extending linearly along its length direction, and a plurality of notches is distributed at equal intervals on the tooth edge, to form teeth spaced apart from each other.

### SUMMARY

The invention is defined in the independent claim and other embodiments are listed in the dependent claims.

The present disclosure is directed to the above problems in the prior art and provides a tool bit for an ultrasonic osteotome comprising a head portion of the tool bit, a bit bar and a bit body, with one end of the bit bar being connected with the head portion of the tool bit, and the other end of the bit bar being connected with the bit body, wherein the head portion of the tool bit has a flat-sheet shape, and the head portion of the tool bit is bent about an axis in an axial direction of the tool bit, with an inner surface and an outer surface thereof having a same radius of curvature, and the head portion of the tool bit is provided with a curved part.

According to the tool bit for an ultrasonic osteotome of the present disclosure, preferably, the curved part is provided on a single side of the head portion of the tool bit.

According to the tool bit for an ultrasonic osteotome of the present disclosure, it is preferable that the curved part is provided with cutter teeth.

According to the tool bit for an ultrasonic osteotome of the present disclosure, preferably, the curved part is provided in distal end of the head portion of the tool bit.

According to the tool bit for an ultrasonic osteotome of the present disclosure, it is preferable that the head portion of the tool bit is provided with cutter teeth on a single side thereof.

According to the tool bit for an ultrasonic osteotome of the present disclosure, preferably, the tool bit for an ultrasonic osteotome is of a hollow structure.

According to the tool bit for an ultrasonic osteotome of the present disclosure, it is preferable that a guide hole is provided at a connection portion between the bit bar and the head portion of the tool bit.

According to the tool bit for an ultrasonic osteotome of the present disclosure, it is preferable that a guide slot is provided in the head portion of the tool bit to communicate with the guide hole.

According to the ultrasonic bone cutter bit of the present disclosure, preferably, the bit bar and the bit body are transitioned by a beveled or curved surface.

According to the tool bit for an ultrasonic osteotome of the present disclosure, preferably, the bit body is provided with a connection mechanism at the other end thereof for connection with an ultrasonic transducer, and the bit body is provided with clamping faces on the outer surface thereof for clamping.

### BRIEF DESCRIPTION OF DRAWINGS

In order to more clearly illustrate the technical solutions in specific embodiments of the present invention or in the prior art, the drawings used in the description of the specific embodiments or the prior art will be briefly described below. Apparently, the drawings described in the following description are only some embodiments of the present disclosure, and those skilled in the art can obtain other drawings based on these drawings without creative work.
Fig. 1 is a schematic structural view of a straight sheet-shaped tool bit for an ultrasonic osteotome in the prior art;
Fig. 2A is a perspective view of a tool bit for an ultrasonic osteotome according to a first embodiment of the present disclosure;
Fig. 2B is a front elevational view of the tool bit for an ultrasonic osteotome according to the first embodiment of the present disclosure;
Fig. 2C is a perspective view of the tool bit for an ultrasonic osteotome according to the first embodiment of the present disclosure viewed from another angle;
Fig. 3A is a perspective view of a tool bit for an ultrasonic osteotome according to the present invention;
Fig. 3B is a front elevational view of the tool bit for an ultrasonic osteotome according to the second embodiment of the present invention;
Fig. 3C is a perspective view of the tool bit for an ultrasonic osteotome according to the second embodiment of the present invention viewed from another angle;
Fig. 4A is a perspective view showing a tool bit for an ultrasonic osteotome having a hollow structure according to an embodiment of the present invention;
Fig. 4B is a front elevational view showing the tool bit for an ultrasonic osteotome having the hollow structure according to the embodiment of the present invention;
Fig. 4C is a perspective view showing the tool bit for an ultrasonic osteotome having the hollow structure according to the embodiment of the present invention viewed from another angle;
Fig. 4D is a bottom plan view showing the tool bit for an ultrasonic osteotome having the hollow structure according to the embodiment of the present invention;
Fig. 4E is a front elevational view showing an internal hollow structure of the tool bit for an ultrasonic osteotome having the hollow structure according to the embodiment of the present invention;
Fig. 4F is a left side view of the tool bit for an ultrasonic osteotome having the hollow structure according to the embodiment of the present invention;
Fig. 5A is a schematic perspective view showing a tool bit for an ultrasonic osteotome having a hollow structure according to another embodiment of the present invention;
Fig 5B is a front elevational view showing the tool bit for an ultrasonic osteotome having the hollow structure according to another embodiment of the present invention;
Fig. 5C is a schematic perspective view showing the tool bit for an ultrasonic osteotome having the hollow structure according to another embodiment of the present invention viewed from another angle;
Fig. 5D a front elevational view showing an internal hollow structure of the tool bit for an ultrasonic osteotome having the hollow structure according to another embodiment of the present invention; and
Fig. 5E is a left side view of the tool bit for an ultrasonic osteotome having the hollow structure according to another embodiment of the present invention.

Reference numerals:
1∼head portion of a tool bit; 2~bit bar; 3~bit body; 4~curved part; 5~cutter teeth; 6~guide hole; 7~guide slot; 8~clamping face.

### DESCRIPTION OF EMBODIMENTS

Exemplary embodiments of the present disclosure will be described hereinafter clearly and completely with reference to the attached drawings. Apparently, the embodiments described herein are only portions of embodiments. The invention is defined by the appended claims.

In the description of the present invention, it is to be noted that the terms of "center", "upper", "lower", "left", "right", "vertical", "horizontal", "internal", "external" and the like simply indicate orientational or positional relationship based on the accompanying drawings and are used only for the purpose of facilitating and simplifying the description of the invention, rather than specifying or implying that any devices or elements indicated must have a certain orientation, be configured or operate in a certain orientation. Therefore, these terms will not be interpreted as limiting the present invention. Further, the terms of "first", "second" and "third" and the like are only used for describing purpose, rather than being interpreted as specifying or implying relative importance.

In the description of the present disclosure, it is to be noted that, unless otherwise specified or defined clearly, the term of "attach", "connect to", "connect with", "couple" and the like should be interpreted broadly. For example, they may refer to fixed connection, or detachable connection, or integral connection; they may refer to mechanical connection, or electrical connection; they may refer to direct connection, or indirect connection through an intermediate agent, or internal communication between two components. For those skilled in the art, the specific meaning of these terms in the present disclosure may be understood in combination with specific situations or contexts.

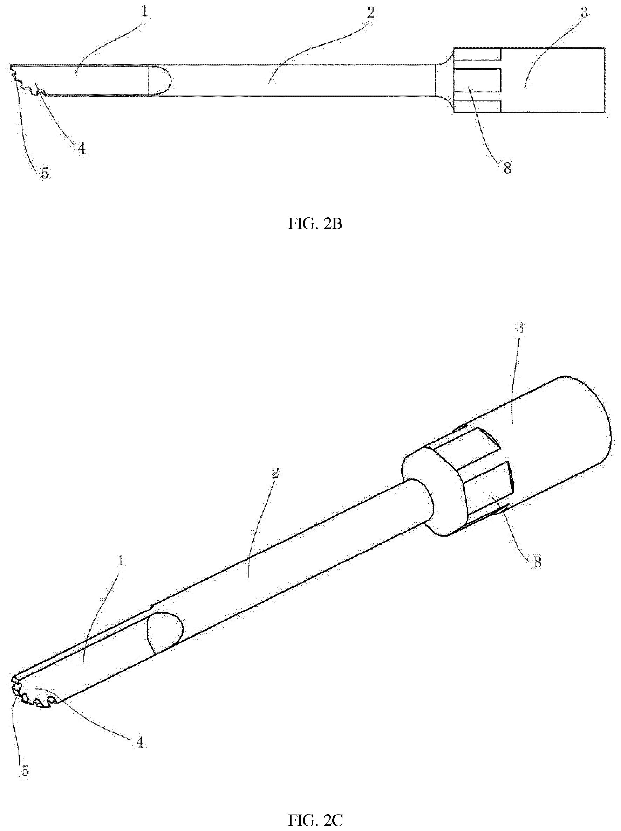

The present disclosure will be further described in detail below by way of specific embodiments in combination with accompanying drawings. Fig. 2A is a schematic perspective view of a tool bit for an ultrasonic osteotome according to a first embodiment of the present disclosure. Fig. 2B is a front view of the tool bit for an ultrasonic osteotome according to the first embodiment of the present disclosure. Fig. 2C is schematic perspective view of the tool bit for an ultrasonic osteotome according to the first embodiment of the present disclosure when viewed from another angle. As shown in Figs. 2A to 2C, the tool bit for an ultrasonic osteotome according to the first embodiment of the present disclosure comprises a head portion 1 of the tool bit, a bit bar 2 and a bit body 3.One end of the bit bar 2 is connected with the head portion 1 of the tool bit, and the other end of the bit bar 2 is connected with the bit body 3. The head portion 1 of the tool bit has a flat-sheet shape or a slightly tapered flat-sheet shape. The head portion 1 of the tool bit is entirely bent inward to a certain angle about an axis in an axial direction of the tool bit, so that the flat-sheet head portion 1 of the tool bit has an inner surface and an arcuate outer surface with a same radius of curvature. That is, the entire sheet-shaped portion of the head portion 1 of the tool bit is curved in a same arcuate degree and has a same thickness. The arcuate shape of the entire head portion 1 of the tool bit constitutes parts of concentric circles. Upon cutting, a diameter of a circle to which the flat-sheet head portion 1 belongs determines a size of a window during operation. The head portion 1 of the tool bit is provided with a curved part 4 on one end thereof. The curved part 4 is formed with cutter teeth 5 thereon. For example, a plurality of arcuate grooves may be provided to form a toothed shape. In the tool bit for an ultrasonic osteotome of the embodiment, as shown in Figs. 2A to 2C, the bit body 3 has a cylindrical shape, and the bit bar 2 has a cylindrical shape. An outer diameter of the bit bar 2 is smaller than an outer diameter of the bit body 3, and there is a smooth transition between the bit bar 2 and the bit body 3 by a beveled or curved surface. The smooth transition structure can effectively prevent stress concentration while avoiding hurt to the patient and the operator from sharp edges and corners. Further, the bit body 3 may be provided with clamping faces 8 for clamping, so that the operator can conveniently clamp the clamping faces 8 by using a clamping tool to screw the tool bit for an ultrasonic osteotome of the present disclosure on a transducer tightly. Preferably, the clamping faces 8 form clamping positions for a hex wrench.

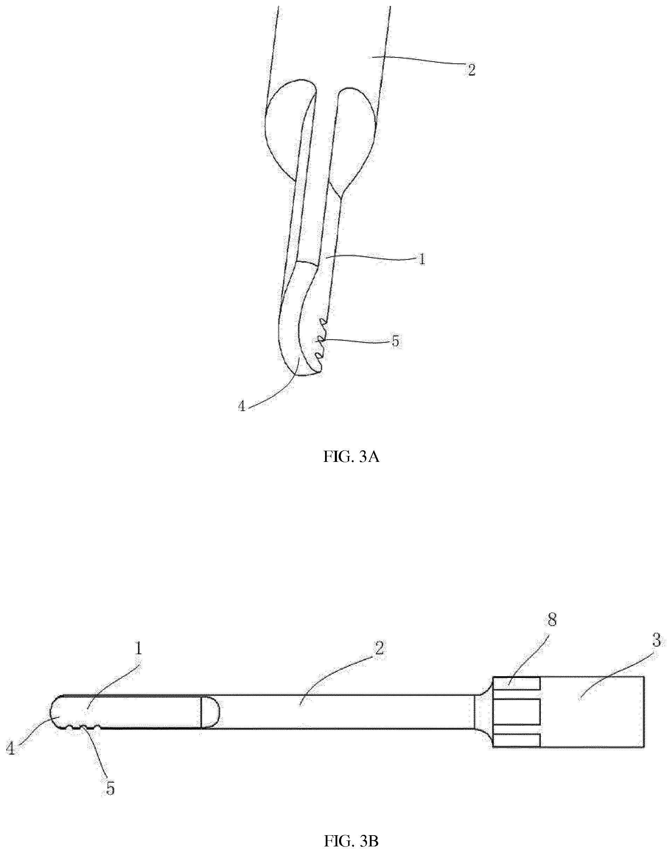

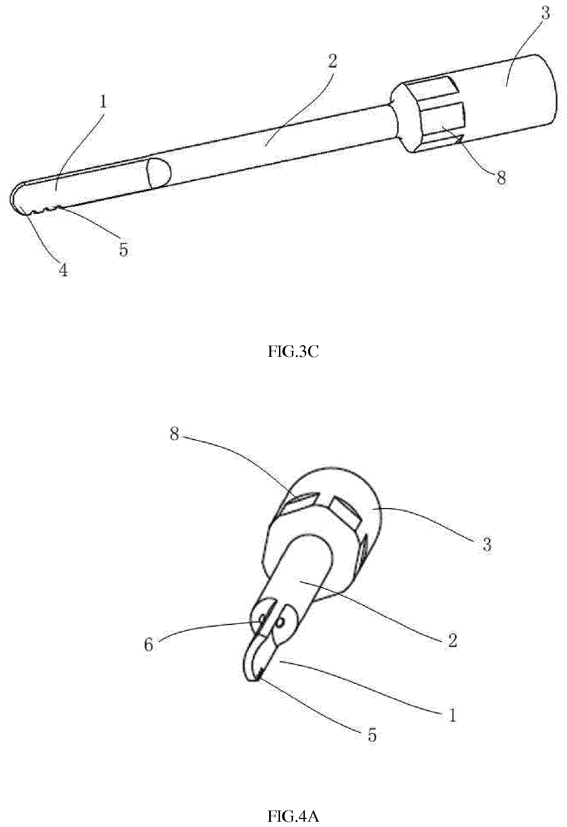

Fig. 3A is a schematic perspective view of a tool bit for an ultrasonic osteotome according to a second embodiment of the present invention, Fig. 3B is a front view of the tool bit for an ultrasonic osteotome according to the second embodiment of the present invention, and Fig. 3C is a schematic perspective view of the tool bit for an ultrasonic osteotome of the second embodiment of the present invention when viewed from another angle. As shown in Figs. 3A to 3C, in the tool bit for an ultrasonic osteotome according to the second embodiment of the present invention, a curved part 4 is formed in distal end of the head portion 1 of the tool bit. The head portion 1 of the tool bit is provided with cutter teeth 5 on a single side thereof. The cutter teeth 5 start from a terminal of the curved part 4. This provides the operator with another way of cutting.

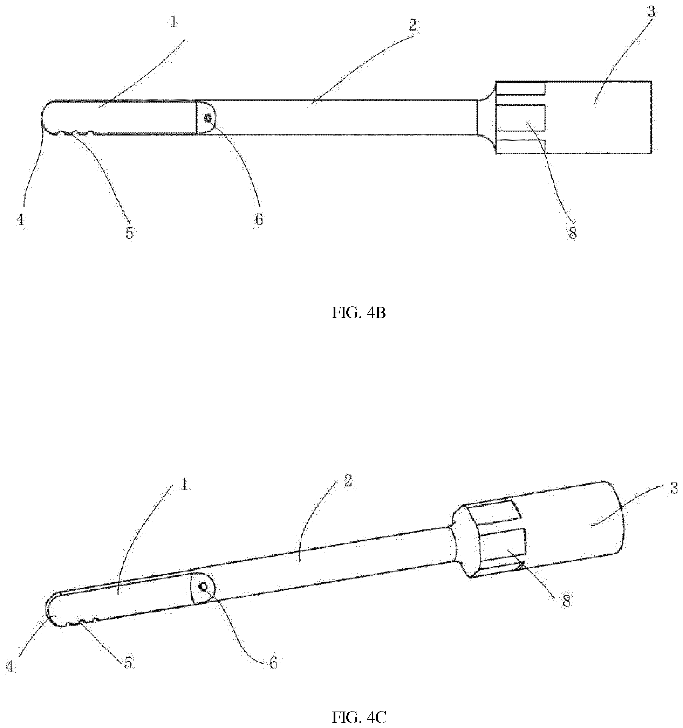

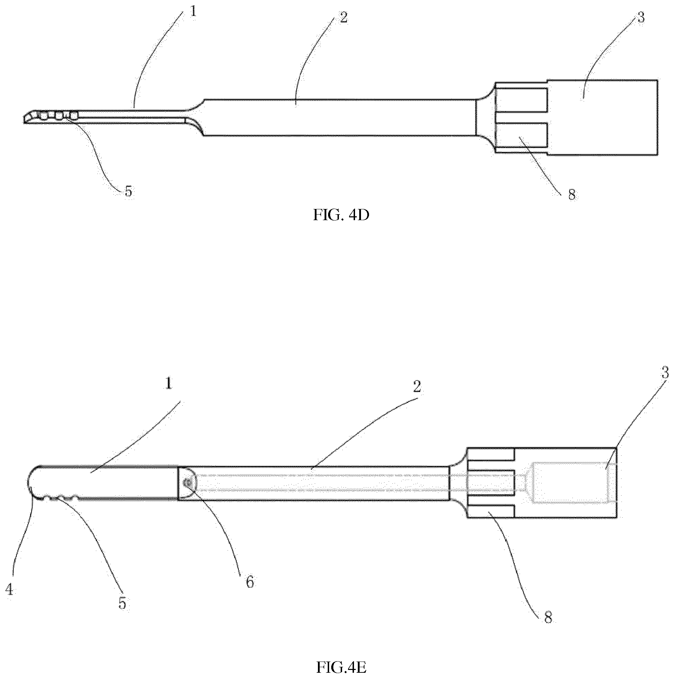

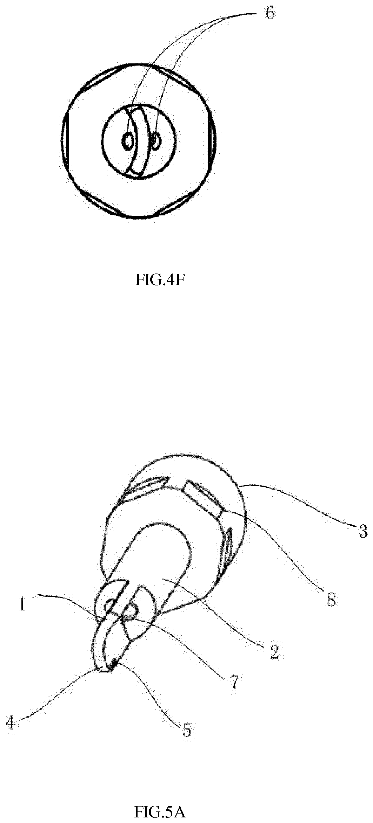

Fig. 4A is a schematic perspective view of a hollow structure of a tool bit for an ultrasonic osteotome according to an embodiment of the present invention. Fig. 4B is a front elevational view showing the tool bit for an ultrasonic osteotome having the hollow structure according to the embodiment of the present invention. Fig. 4C is a schematic perspective view showing the tool bit for an ultrasonic osteotome having the hollow structure according to the embodiment of the present invention viewed from another angle. Fig. 4D is a bottom plan view showing the tool bit for an ultrasonic osteotome having the hollow structure according to the embodiment of the present invention. Fig. 4E is a front elevational view showing an internal hollow structure of the tool bit for an ultrasonic osteotome having the hollow structure according to the embodiment of the present invention. Fig. 4F is a left side view of the tool bit for an ultrasonic osteotome having the hollow structure according to the embodiment of the present invention. As shown in Figs. 4A to 4F, as a modification of the first embodiment of the present invention, a tool bit for an ultrasonic osteotome of the present invention may be provided with a through hole at a center of the tool bit to form a hollow structure, and a guide hole 6 may be provided at a connection portion between the bit bar 2 and the head portion 1 of the tool bit. There may be two guide holes 6 provided at each of an inner surface and an outer surface of the head portion 1 of the tool bit, respectively. The guide holes 6 can ensure to guide water flow to a surgical cutting surface of the head portion 1 of the tool bit, and also have a cooling effect for the tool bit itself. During the operation, the entire head portion of the tool bit and the contacted tissue are completely exposed to the perfusate for cleaning and cooling in real time, ensuring that the excised bone fragments are immediately discharged, and the visual field at the incision is clear and clean so that the tissue to be retained is protected from being damaged. This can further reduce the risk of operation and improve the safety and success rate of surgery. The tool bit of an ultrasonic osteotome with a hollow structure can also reduce the weight of the tool bit to a certain extent, so that the tool bit is smaller and lighter in structure, more labor-saving for gripping, and more convenient for operation.

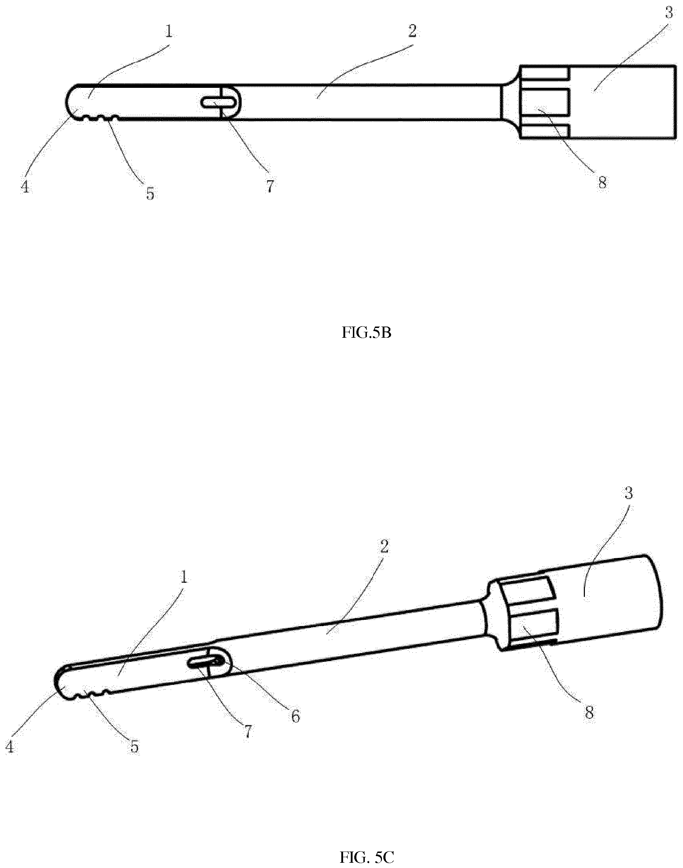

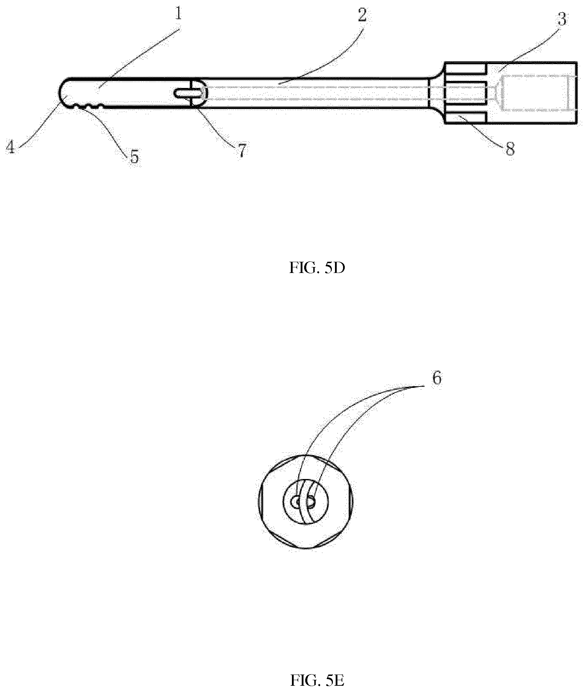

Fig. 5A is a schematic perspective view showing a tool bit for an ultrasonic osteotome having a hollow structure according to another embodiment of the present invention. Fig 5B is a front elevational view showing the tool bit for an ultrasonic osteotome having the hollow structure according to another embodiment of the present invention. Fig. 5C is a schematic perspective view showing the tool bit for an ultrasonic osteotome having the hollow structure according to another embodiment of the present invention viewed from another angle. Fig. 5D is a front elevational view showing an internal hollow structure of the tool bit for an ultrasonic osteotome having the hollow structure according to another embodiment of the present invention. Fig. 5E is a left side view of the tool bit for an ultrasonic osteotome having the hollow structure according to another embodiment of the present invention. As shown in Figs. 5A to 5E, the tool bit for an ultrasonic osteotome of the present embodiment has a hollow structure by providing a through hole in a center of the tool bit. A guide hole 6 is provided at a connection portion between the head portion 1 of the tool bit and the bit bar 2, and a guide slot 7 is provided in the head portion 1 of the tool bit. The guide slot 7 may be provided only in a part of the head portion 1 of the tool bit, as shown in Figs. 5B to 5D. The guide slot 7 can also be extended all the way to the front top end of the head portion 1 of the tool bit. The presence of the guide slot 7 can ensure that a greater amount of water flow is guided to a surgical cutting portion at the head portion of the tool bit, providing more adequate cooling of the surgical site of the tool bit. During the operation, the entire head portion of the tool bit and the contacted tissue are completely exposed to a perfusate for cleaning and cooling in real time, ensuring that the bone excised fragments can be discharged more immediately, and the visual field at the incision is clearer and cleaner, and further improving the cleaning and cooling effects in the operation.

In the tool bit for an ultrasonic osteotome of embodiments of the present invention, a connection mechanism may be provided on the bit body 3. Preferably, the connection mechanism is a threaded structure, which may be an external thread or an internal thread. Of course, the connection mechanism is not limited to the threaded structure, any connection mechanism capable of fixing the tool bit for an ultrasonic osteotome of the present invention to a transducer in a detachable manner can be provided on the bit body of the tool bit for an ultrasonic osteotome of the present invention. When the connection thread on the bit body of the tool bit for an ultrasonic osteotome of the present invention is connected to a specific ultrasonic transducer and tightened with a corresponding wrench, and then the ultrasonic transducer is connected to a specific ultrasonic main machine, it is ready to work.

Compared with the prior art, the embodiments of the invention have the following advantages: the tool bit for an ultrasonic osteotome of the invention can concentrate all the energy generated by the ultrasonic transducer on the front end portion of the tool bit (the most effective working part), so that the front end portion of the tool bit has the strongest energy output for the strongest working effect. Since a back side portion of a main cutting portion of the head portion of the tool bit, i.e., a back side portion formed with the cutter teeth, adopts a planar or an arcuate structure, it can effectively avoid damage to the spinal cord by scratching when a cutting operation is performed by bypassing the spinal cord, thereby improving the safety of the operation. When the tool bit for an ultrasonic osteotome is used for osteotomy, it can conveniently and quickly cut a hole in the vertebral plate or other bone tissue, thus the tool bit for an ultrasonic osteotome is suitable for minimally invasive surgery in orthopedic surgery, and can reduce operation time.

It should be noted that the above embodiments are only used to describe the concept of the present invention, rather than limiting the present invention. Although detailed descriptions of the invention are made with reference to the above embodiments, it would be appreciated by those skilled in the art that various changes or modifications to the above embodiments may be made to portion of or all features in those embodiments may be made. The invention is defined by the appended claims.

## Claims

1. A tool bit for an ultrasonic osteotome comprising a head portion (1) of the tool bit, a bit bar (2) and a bit body (3), with one end of the bit bar (2) being connected with the head portion (1) of the tool bit, and the other end of the bit bar (2) being connected with the bit body (3), wherein the head portion (1) of the tool bit has a flat-sheet shape, and the head portion (1) of the tool bit is bent about a longitudinal axis in an axial direction of the tool bit, with an inner surface and an outer surface of the flat-sheet head portion (1) of the tool bit having a same radius of curvature, and the head portion (1) of the tool bit is provided with a curved part (4),
**characterized in that** the curved part (4) is provided at the distal end of the head portion (1) of the tool bit, and the head portion (1) of the tool bit is provided with cutter teeth (5) on a single side thereof, wherein the cutter teeth (5) start from a terminal of the curved part (4).

2. The tool bit for an ultrasonic osteotome of claim 1, wherein the tool bit for an ultrasonic osteotome is of a hollow structure.

3. The tool bit for an ultrasonic osteotome of claim 2, wherein a guide hole (6) is provided at a connection portion between the bit bar (2) and the head portion (1) of the tool bit.

4. The tool bit for an ultrasonic osteotome of claim 3, wherein a guide slot (7) is provided in the head portion (1) of the tool bit to communicate with the guide hole (6).

5. The tool bit for an ultrasonic osteotome of claim 1, 3 or 4, wherein the bit bar (2) and the bit body (3) are transitioned by a beveled or curved surface.

6. The tool bit for an ultrasonic osteotome of claim 1, 3 or 4, wherein the bit body (3) is provided with a connection mechanism at the other end thereof for connection with an ultrasonic transducer, and the bit body (3) is provided with clamping faces (8) on an outer surface thereof for clamping.

## Patentansprüche

1. Werkzeugeinsatz für ein Ultraschallosteotom, der einen Kopfabschnitt (1) des Werkzeugeinsatzes, eine Werkzeugstange (2) und einen Werkzeugkörper (3) umfasst, wobei ein Ende der Werkzeugstange (2) mit dem Kopfabschnitt (1) des Werkzeugeinsatzes verbunden ist und das andere Ende der Werkzeugstange (2) mit dem Werkzeugkörper (3) verbunden ist, wobei der Kopfabschnitt (1) des Werkzeugeinsatzes flachblattförmig ist, und der Kopfabschnitt (1) des Werkzeugeinsatzes um eine Längsachse in einer axialen Richtung des Werkzeugeinsatzes gebogen ist, wobei eine innere Oberfläche und eine äußere Oberfläche des flachblattförmigen Kopfabschnitts (1) des Werkzeugeinsatzes einen gleichen Krümmungsradius aufweisen und der Kopfabschnitt (1) des Werkzeugeinsatzes mit einem gekrümmten Teil (4) versehen ist,
**dadurch gekennzeichnet, dass** der gekrümmte Teil (4) am distalen Ende des Kopfabschnitts (1) des Werkzeugeinsatzes vorgesehen ist und der Kopfabschnitt (1) des Werkzeugeinsatzes mit Schneidzähnen (5) auf einer einzigen Seite davon versehen ist, wobei die Schneidzähne (5) von einem Ende des gekrümmten Teils (4) ausgehen.

2. Werkzeugeinsatz für ein Ultraschallosteotom nach Anspruch 1, wobei der Werkzeugeinsatz für ein Ultraschallosteotom eine hohle Struktur aufweist.

3. Werkzeugeinsatz für ein Ultraschallosteotom nach Anspruch 2, wobei ein Führungsloch (6) an einem Verbindungsabschnitt zwischen der Werkzeugstange (2) und dem Kopfabschnitt (1) des Werkzeugaufsatzes vorgesehen ist.

4. Werkzeugeinsatz für ein Ultraschallosteotom nach Anspruch 3, wobei ein Führungsschlitz (7) im Kopfabschnitt (1) des Werkzeugeinsatzes vorgesehen ist, um mit dem Führungsloch (6) in Verbindung zu stehen.

5. Werkzeugeinsatz für ein Ultraschallosteotom nach Anspruch 1, 3 oder 4, wobei die Werkzeugstange (2) und der Werkzeugkörper (3) durch eine abgeschrägte oder gekrümmte Oberfläche ineinander übergehen.

6. Werkzeugeinsatz für ein Ultraschallosteotom nach Anspruch 1, 3 oder 4, wobei der Werkzeugkörper (3) an seinem anderen Ende mit einem Verbindungsmechanismus zur Verbindung mit einem Ultraschallwandler versehen ist, und der Werkzeugkörper (3) an seiner Außenfläche mit Klemmflächen (8) zum Festklemmen versehen ist.

## Revendications

1. Tête d'outil pour un ostéotome à ultrasons comprenant une partie frontale (1) de la tête d'outil, une barre de tête (2) et un corps de tête (3), une extrémité de la barre de tête (2) étant reliée à la partie frontale (1) de la tête d'outil, et l'autre extrémité de la barre de tête (2) étant reliée au corps de tête (3), dans lequel la partie frontale (1) de la tête d'outil a la forme d'une feuille plate, et la partie frontale (1) de la tête d'outil est pliée autour d'un axe longitudinal dans une direction axiale de la tête d'outil, une surface intérieure et une surface extérieure de la partie frontale (1) de la tête d'outil ayant un même rayon de courbure, et la partie frontale (1) de la tête d'outil est pourvue d'une partie courbée (4),
**caractérisé par le fait que** la partie courbée (4) se trouve à l'extrémité distale de la partie frontale (1) de la tête d'outil, et que la partie frontale (1) de la tête d'outil est pourvue de dents de coupe (5) sur un seul de ses côtés, les dents de coupe (5) commençant à partir d'une extrémité de la partie courbée (4).

2. Tête d'outil pour un ostéotome à ultrasons selon la revendication 1, dans lequel la tête d'outil pour un ostéotome à ultrasons est de structure creuse.

3. Tête d'outil pour un ostéotome à ultrasons selon la revendication 2, dans lequel un trou de guidage (6) est prévu dans une partie de connexion entre la barre de tête (2) et la partie frontale (1) de la tête d'outil.

4. Tête d'outil pour un ostéotome à ultrasons selon la revendication 3, dans lequel une fente de guidage (7) est prévue dans la partie frontale (1) de la tête d'outil pour communiquer avec le trou de guidage (6).

5. Tête d'outil pour un ostéotome à ultrasons selon la revendication 1, 3 ou 4, dans lequel la barre de tête (2) et le corps de tête (3) sont séparés par une surface biseautée ou incurvée.

6. Tête d'outil pour un ostéotome à ultrasons selon la revendication 1, 3 ou 4, dans lequel le corps de tête (3) est pourvu d'un mécanisme de connexion à l'autre extrémité pour la connexion avec un transducteur à ultrasons, et le corps de tête (3) est pourvu de faces de serrage (8) sur une surface extérieure pour le serrage.
